# EUROPEAN PATENT APPLICATION

(11) **EP 3 187 115 A1**
(43) Date of publication of application: **05.07.2017**
(21) Application number: 15835927.3
(22) Date of filing: 20.08.2015
(51) Int. Cl.: A61B 8/13

(54) **PHOTOACOUSTIC IMAGING DEVICE**

(30) Priority: 27.08.2014 JP 2014172786; 27.08.2014 JP 2014172771
(71) Applicant: PreXion Corporation, Chiyoda-ku Tokyo 101-0041 (JP)
(72) Inventor: NAKATSUKA, Hitoshi, Tokyo 101-0041 (JP); AGANO, Toshitaka, Tokyo 101-0041 (JP)
(74) Representative: Grünecker Patent- und Rechtsanwälte PartG mbB
(86) International application number: PCT/JP2015/073442
(87) International publication number: WO 2016/031687

(57) **Abstract**

This photoacoustic imaging device (100) comprises: a display unit (40) that rewrites a screen (40a) at a predetermined refresh rate (RC) and that displays an image on the screen; and a control unit (30) that performs the acquisition of a detection signal and irradiation with light by a semiconductor light-emitting-element light-source unit at a sampling cycle (SC) shorter than the cycle of the predetermined refresh rate, and that creates an image to be displayed on the display unit from the detection signal detected according to the sampling cycle.

## Description

### Technical Field

The present invention relates to a photoacoustic imaging apparatus, specifically, to a photoacoustic imaging apparatus including a display portion.

### Background Art

A photoacoustic imaging apparatus including a display portion has conventionally been known. Such a photoacoustic imaging apparatus is disclosed in Japanese Patent Laying-Open No. 2012-250019, for example.

Japanese Patent Laying-Open No. 2012-250019 discloses a photoacoustic imaging apparatus including: a light source that generates pulsed light at certain light emission frequencies (20 Hz and 40 Hz); a receiver that detects an acoustic wave generated by a detection target in a test object when the detection target absorbs the pulsed light and converts the detected acoustic wave to a receiving signal; a signal processing portion that generates an image by executing averaging processing on the receiving signal; and a monitor (display portion) on which the image is displayed. A screen of this monitor (display portion) is generally updated at a certain refresh rate (60 Hz, for example).

### Citation List

### Patent Literature

Patent Literature 1: Japanese Patent Laying-Open No. 2012-250019

### Summary of Invention

### Technical Problem

In the above-described photoacoustic imaging apparatus described in Japanese Patent Laying-Open No. 2012-250019, light emission frequencies (20 Hz and 40 Hz) are lower than a general refresh rate (60 Hz, for example). This makes a light emission cycle (a sampling cycle) longer than a cycle of updating the screen of the display portion (a cycle of a refresh rate). Hence, in some cases, both light emission and acquisition of a detection signal cannot be achieved within a cycle of updating the screen. In these cases, a detection signal is not acquired within the cycle of updating the screen. This makes it impossible for the signal processing portion to generate a new image within the cycle of updating the screen, causing an unfavorable issue of failing to update the screen (displaying one image continuously). In this case, the cycle of updating the screen is substantially extended and this is considered to cause a problem of awkward motion of moving images displayed on the screen (loss of smoothness).

The present invention has been made to solve the above-described problem. It is one object of the present invention to provide a photoacoustic imaging apparatus capable of suppressing awkward motion of moving images displayed on a screen.

### Solution to Problem

A photoacoustic imaging apparatus according to one aspect of the present invention includes: a semiconductor light-emitting element light source portion; a detecting portion that detects an acoustic wave generated from a detection target in a test object when the detection target absorbs light emitted from the semiconductor light-emitting element light source portion and outputs a detection signal; a display portion having a screen to be updated at a certain refresh rate and on which an image is to be displayed; and a control portion that makes the semiconductor light-emitting element light source portion emit light in a sampling cycle shorter than a cycle of the certain refresh rate, acquires the detection signal in the sampling cycle, and generates an image to be displayed on the display portion using the detection signal detected in the sampling cycle.

As described above, in the photoacoustic imaging apparatus according to the aforementioned aspect of the present invention, the control portion is provided that makes the semiconductor light-emitting element light source portion emit light in the sampling cycle shorter than the cycle of the certain refresh rate, acquires the detection signal in the sampling cycle, and generates an image to be displayed on the display portion using the detection signal detected in the sampling cycle. As the sampling cycle is set to be shorter than the cycle of the certain refresh rate, a detection signal can be acquired within the cycle of the certain refresh rate. Thus, the detection signal acquired within the cycle of the certain refresh rate can be used for generating an image. This can easily reduce the likelihood of failing to update the screen of the display portion (displaying one image continuously) due to failing to acquire a detection signal during a cycle of updating the screen (cycle of the certain refresh rate). This can easily make it less likely that the cycle of updating the screen will be substantially extended. As a result, awkward motion of moving images displayed on the screen (loss of smoothness) can be suppressed. Further, provision of the semiconductor light-emitting element light source portion can shorten the sampling cycle easily, compared to use of a solid-laser light source portion having difficultly in shortening a light emission cycle (sampling cycle).

In the photoacoustic imaging apparatus according to the aforementioned aspect, the control portion is preferably configured to generate an image to be displayed on the display portion by taking an arithmetic average of the detection signals detected in the sampling cycles. In this configuration, an arithmetic average of the detection signals detected in the sampling cycles is taken, so that an image can be generated with an increased S/N ratio (signal-to-noise ratio).

In the aforementioned configuration of generating an image to be displayed on the display portion by taking an arithmetic average of the detection signals, the sampling cycle preferably has a length that allows the semiconductor light-emitting element light source portion to emit light several times while allowing the detection signal to be acquired several times within the cycle of the certain refresh rate. In this configuration, the several detection signals can be acquired within the cycle of the certain refresh rate. This can more easily reduce the likelihood of failing to update the screen of the display portion due to failing to acquire a detection signal during a cycle of updating the screen (cycle of the certain refresh rate). As a result, awkward motion of moving images displayed on the screen can be suppressed more easily.

In the aforementioned configuration of generating an image to be displayed on the display portion by taking an arithmetic average of the detection signals, the control portion is preferably configured to generate an image to be displayed on the display portion by taking an arithmetic average of the detection signals with the number of additions that makes a value obtained by multiplying the sampling cycle and the number of additions during taking of an arithmetic average exceed the cycle of the certain refresh rate. This configuration can reduce the likelihood of the occurrence of a detection signal out of the acquired detection signals and not to be reflected in generation of an image, compared to a case where a value obtained by multiplying the sampling cycle and the number of additions during taking of an arithmetic average does not exceed the cycle of the certain refresh rate.

In the aforementioned configuration of generating an image to be displayed on the display portion by taking an arithmetic average of the detection signals, the control portion is preferably configured to generate an image to be displayed on the display portion by taking a moving average as an arithmetic average. This configuration makes it possible to generate an image in such a manner as to smoothen a motion (difference) between individual generated images, unlike the case of taking a average as an arithmetic average. Thus, moving images formed of the individual images can be displayed smoothly. This configuration works effectively, particularly in displaying moving images of the inside of the test object to change constantly such as a human body.

In this case, the control portion is preferably configured to take a moving average each time the detection signal is acquired. In this configuration, averaging processing is executed on every occasion on each detection signal by taking a moving average of detection signals, so that averaging processing for generating an image can be executed reliably at each certain refresh rate. As a result, an image can be generated reliably during update of the screen, making it possible to reliably reduce the likelihood of failing to update the screen. Thus, awkward motion of moving images displayed on the screen can be suppressed reliably.

In the aforementioned configuration of generating an image to be displayed on the display portion by taking an arithmetic average of the detection signals, the control portion is preferably configured to generate an image to be displayed on the display portion by taking a average as an arithmetic average. This configuration makes it possible to reduce the number of detection signals to be stored in a memory, compared to taking a moving average as an arithmetic average. This achieves a corresponding saving of the volume of the memory.

In the aforementioned configuration of generating an image to be displayed on the display portion by taking an arithmetic average of the detection signals, the control portion is preferably configured to generate an image to be displayed on the display portion by taking an arithmetic average of the detection signals with the number of additions that makes a value obtained by multiplying the sampling cycle and the number of additions during taking of an arithmetic average 125 msec or less. In this configuration, the redundancy of averaging time can be suppressed. This can suppress awkward motion of moving images formed of individual images due to redundancy of averaging time.

In the photoacoustic imaging apparatus according to the aforementioned aspect, the control portion is preferably configured to generate a average signal by taking a average of detection signals from a detection signal acquired first to a detection signal acquired last within the cycle of the certain refresh rate and to generate an image to be displayed on the display portion based on the average signal. In this configuration, all detection signals acquired within the cycle of the certain refresh rate can be used for generating an image. This can reduce the likelihood of the occurrence of a detection signal out of acquired detection signals and not to be reflected in an image to be displayed on the screen of the display portion. As a result, the acquired detection signals can be used efficiently for generating an image.

In the aforementioned configuration of generating an image to be displayed on the display portion based on the average signal, the control portion is preferably configured to generate an image to be displayed on the display portion by defining the average signal generated by taking a average as one unit and acquiring average signals as a plurality of units, and by taking an arithmetic average of the acquired average signals as the plurality of units. This configuration achieves generation of an image by executing averaging processing on more detection signals, so that the image can be generated with an increased S/N ratio (signal-to-noise ratio). As a result, a clear image can be generated through efficient use of acquired detection signals.

In this case, the control portion is preferably configured to generate an image to be displayed on the display portion by taking a moving average as an arithmetic average. In this configuration, while the average signals are generated by taking a average, a moving average of these average signals is taken. This makes it possible to generate an image in such a manner as to smoothen a motion (difference) between individual images. Thus, moving images formed of the individual images can be displayed smoothly while acquired detection signals are used efficiently. This configuration of taking a moving average works effectively, particularly in displaying moving images of the inside of the test object to change constantly such as a human body.

In the aforementioned configuration of taking a moving average as an arithmetic average, the control portion is preferably configured to take a moving average each time the average signal is acquired. In this configuration, averaging processing is executed on every occasion on each average signal by taking a moving average of the average signals, so that averaging processing for generating an image can be executed reliably at each certain refresh rate. As a result, an image can be generated reliably during update of the screen, making it possible to reliably reduce the likelihood of failing to update the screen. In this way, while an image is generated by efficiently using acquired detection signals, awkward motion of moving images displayed on the screen can be suppressed reliably.

In the aforementioned configuration of generating an image to be displayed on the display portion based on the average signal, the control portion is preferably configured to generate an image to be displayed on the display portion by taking a average as an arithmetic average. This configuration makes it possible to reduce the number of detection signals to be stored in the memory, compared to taking a moving average as an arithmetic average. This achieves a corresponding saving of the volume of the memory.

In the aforementioned configuration of taking an arithmetic average of the average signals as the plurality of units, the control portion is preferably configured to acquire the average signals as the plurality of units in such a manner that a value obtained by multiplying the sampling cycle and the number of detection signals to be subjected to averaging processing by taking of an arithmetic average is 125 msec or less. In this configuration, the redundancy of averaging time can be suppressed. This can suppress awkward motion of moving images formed of individual images due to redundancy of averaging time.

In the photoacoustic imaging apparatus according to the aforementioned aspect, the control portion is preferably configured to acquire the detection signal in a certain sampling cycle, and the control portion is preferably configured to synchronize timing of start of the cycle of the certain refresh rate and timing of start of the certain sampling cycle with each other for each cycle of the certain refresh rate. In this configuration, non-uniformity of the number of detection signals between cycles of the certain refresh rate can be less likely to occur in each cycle of the certain refresh rate. In this way, the number of detection signals to form the average signal can be less likely to vary between the cycles of the certain refresh rate, making it possible to reduce the likelihood of the occurrence of a difference in an image quality between individual images to be generated.

In this case, the control portion is preferably configured to synchronize timing of start of the cycle of the certain refresh rate and timing of start of the certain sampling cycle with each other by changing the length of a last sampling cycle within the certain refresh cycle. In this configuration, timing of start of the cycle of the certain refresh rate and timing of start of the certain sampling cycle can be synchronized with each other only by changing the length of the last sampling cycle within the certain sampling cycle. As a result, timing of start of the cycle of the certain refresh rate and timing of start of the certain sampling cycle can be synchronized with each other easily.

In the photoacoustic imaging apparatus according to the aforementioned aspect, the control portion is preferably configured to define the average signal generated by taking a average as one unit and acquire the average signal as one unit or acquire the average signals as a plurality of units in such a manner that the number of the detection signals reaches a certain number or more, and to generate an image to be displayed on the display portion based on the acquired average signal as one unit or the acquired average signals as the plurality of units. This configuration makes it possible to execute averaging processing reliably using the detection signals of the certain number or more, so that an S/N ratio (signal-to-noise ratio) can be increased reliably during generation of an image. As a result, a clear image can be generated reliably.

In the photoacoustic imaging apparatus according to the aforementioned aspect, the semiconductor light-emitting element light source portion preferably includes a light-emitting diode element as the semiconductor light-emitting element. This configuration of using the light-emitting diode element of relatively low power consumption can reduce power consumption.

In the photoacoustic imaging apparatus according to the aforementioned aspect, the semiconductor light-emitting element light source portion preferably includes a semiconductor laser element as the semiconductor light-emitting element. In this configuration, a laser beam of relatively high directivity can be emitted to the test object, compared to using the light-emitting diode element. Thus, much of the beam from the semiconductor laser element can be applied reliably to the test object.

In the photoacoustic imaging apparatus according to the aforementioned aspect, the semiconductor light-emitting element light source portion preferably includes an organic light-emitting diode element as the semiconductor light-emitting element. This configuration of using the organic light-emitting diode element capable of being reduced in thickness easily facilitates size reduction of the semiconductor light-emitting element light source portion.

### Advantageous Effects of Invention

As described above, according to the present invention, a photoacoustic imaging apparatus capable of suppressing awkward motion of moving images displayed on a screen can be provided.

### Brief Description of Drawings

FIG. 1 is a view showing the overall configuration of a photoacoustic imaging apparatus according to a first embodiment and a second embodiment of the present invention.
FIG. 2 is a schematic view showing a state of measuring an acoustic wave by the photoacoustic imaging apparatus according to the first and second embodiments of the present invention.
FIG. 3 is a view for explaining a case where the number of additions during averaging processing by taking of a moving average is small.
FIG. 4 is a view for explaining a case where the number of additions during averaging processing by taking of a moving average is large.
FIG. 5 is a view for explaining averaging processing by taking of a average by a photoacoustic imaging apparatus according to the second embodiment of the present invention.
FIG. 6 is a view showing the overall configuration of a photoacoustic imaging apparatus according to a third embodiment, a fourth embodiment, and a fifth embodiment of the present invention.
FIG. 7 is a schematic view showing a state of measuring an acoustic wave by the photoacoustic imaging apparatus according to the third embodiment of the present invention.
FIG. 8 is a view for explaining a average signal generated by the photoacoustic imaging apparatus according to the third embodiment of the present invention.
FIG. 9 is a view for explaining generation of an image using a average signal by the photoacoustic imaging apparatus according to the third embodiment of the present invention.
FIG. 10 is a view for explaining generation of an image using a average signal by the photoacoustic imaging apparatus according to the fourth embodiment of the present invention.
FIG. 11 is a view for explaining a average signal generated by the photoacoustic imaging apparatus according to the fifth embodiment of the present invention.

### Description of Embodiments

Embodiments of the present invention will be described below by referring to the drawings.

### (First Embodiment)

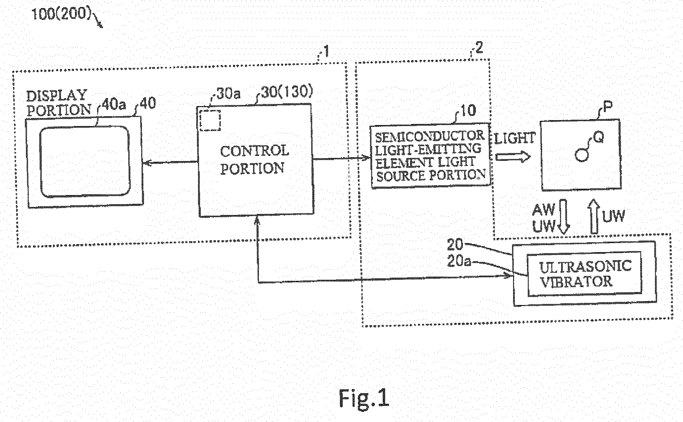

The configuration of a photoacoustic imaging apparatus 100 according to a first embodiment of the present invention will be described first by referring to FIGS. 1, 2, and 4.

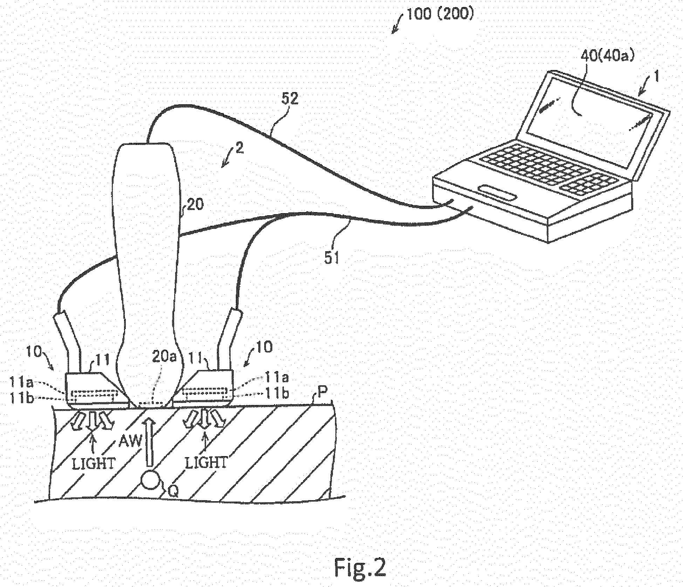

As shown in FIG. 1 and 2, the photoacoustic imaging apparatus 100 according to the first embodiment of the present invention includes an apparatus body 1 and a probe portion 2. The apparatus body 1 and the probe portion 2 are connected through a wire 51 and a wire 52. The apparatus body 1 includes a control portion 30 and a display portion 40. The probe portion 2 includes a semiconductor light-emitting element light source portion 10 (hereinafter called a light source portion 10) and a detecting portion 20.

As shown in FIGS. 1 and 2, the light source portion 10 includes two light sources 11 and is configured to emit pulsed light for measurement from each of the two light sources 11 toward a test object P. Each of the two light sources 11 is connected through the wire 51 to the control portion 30 and is configured to receive power and a control signal supplied from the control portion 30 through the wire 51.

The two light sources 11 are arranged near the detecting portion 20 and on opposite sides of the detecting portion 20 so as to sandwich the detecting portion 20 therebetween. In this way, the two light sources 11 are configured to emit pulsed light toward the test object P from different positions.

As shown in FIG. 1, each of the two light sources 11 includes a light source substrate 11a and a semiconductor light-emitting element 11b. For example, a light-emitting diode element, a semiconductor laser element, or an organic light-emitting diode element is applicable as the semiconductor light-emitting element 11b. The light source substrate 11a has a lower surface holding a plurality of semiconductor light-emitting elements 11b mounted in an array pattern. The light source substrate 11a has a light source drive circuit and is configured to make the semiconductor light-emitting element 11b emit a pulse based on a control signal output from the control portion 30.

The two semiconductor light-emitting elements 11b are both configured to generate light of a measurement wavelength in an infrared region suitable for measurement of the test object P such as a human body (light having a center wavelength from about 700 to about 1000 nm, for example). The two semiconductor light-emitting elements 11b may be configured to generate light of different measurement wavelengths or light of the substantially same measurement wavelength. A measurement wavelength may be determined properly in a manner that depends on an intended detection target to be detected.

As shown in FIGS. 1 and 2, the detecting portion 20 is an ultrasonic probe and connected to the control portion 30 through the wire 52. The detecting portion 20 includes an ultrasonic vibrator 20a. In the detecting portion 20, the ultrasonic vibrator 20a includes a plurality of ultrasonic vibrators 20a arranged in an array pattern. The detecting portion 20 is configured to detect an acoustic wave (ultrasonic wave) AW in response to vibration of the ultrasonic vibrator 20a caused by an acoustic wave generated from a detection target Q in the test object P when the detection target Q absorbs pulsed light emitted from the light source portion 10. The detecting portion 20 is configured to be capable of generating an ultrasonic wave UW by making the ultrasonic vibrator 20a vibrate based on a control signal output from the control portion 30. The detecting portion 20 is also configured to detect the ultrasonic wave UW in response to vibration of the ultrasonic vibrator 20a caused by this ultrasonic wave UW reflected in the test object P. The detecting portion 20 is configured to output a detection signal corresponding to the detected acoustic wave AW or the detected ultrasonic wave UW to the control portion 30 through the wire 52.

In this description, for the convenience of explanation, an ultrasonic wave generated by absorption of pulsed light by the detection target Q in the test object P will be called an "acoustic wave," an ultrasonic wave generated by the ultrasonic vibrator 20a and reflected in the test object P will be called an "ultrasonic wave," and the "acoustic wave" and the "ultrasonic wave" will be described distinctively.

The control portion 30 includes a CPU and a storage portion 30a such as a ROM or a RAM. As shown in FIG. 1, the control portion 30 is configured to form an image of the inside of the test object P based on a detection signal output from the detecting portion 20. More specifically, the control portion 30 is configured to generate a photoacoustic image based on a detection signal resulting from the acoustic wave AW and to generate an ultrasonic image based on a detection signal resulting from the ultrasonic wave UW. The control portion 30 is configured to be capable of forming an image of various types of information in the test object P by synthesizing the photoacoustic image and the ultrasonic image. Generation of the photoacoustic image resulting from the acoustic wave AW will be described in detail later.

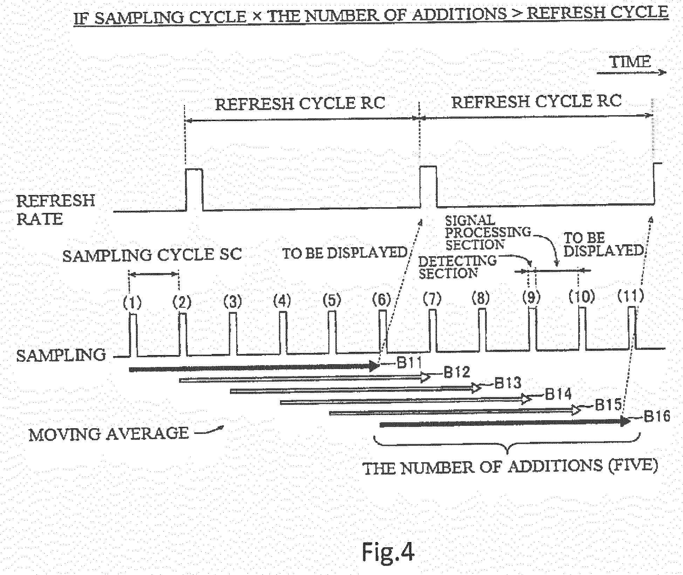

As shown in FIG. 1, the display portion 40 is formed of a general liquid crystal type monitor or a scanning type monitor. The display portion 40 has a screen 40a and is configured to display an image such as an image of the inside of the test object P on the screen 40a formed by the control portion 30. As shown in FIG. 4, the display portion 40 is configured in such a manner that the screen 40a is updated at a certain refresh rate (frequency). The certain refresh rate to be applied is generally about 50 Hz or more (about 50 Hz, about 60 Hz, or about 120 Hz, for example), and these values are applicable as the certain refresh rate.

The refresh rate mentioned herein is a value indicating the number of times the screen 40a is refreshed (updated) per unit time (one second, for example). This means that, if the refresh rate is about 60 Hz, the screen 40a is refreshed (updated) about 60 times in one second. In other words, this means that the screen 40a is refreshed (updated) in each cycle of a refresh rate (hereinafter called a refresh cycle) RC of about 16.7 msec (milliseconds). In FIG. 4, timing of refresh (update) of the screen 40a is schematically shown by a substantially rectangular pulse wave. In the case of a liquid crystal type monitor, for example, the screen 40a is refreshed at the time of rising of a substantially rectangular pulse wave. In the case of a scanning type monitor, for example, the monitor is scanned within the refresh cycle RC to refresh the screen 40a.

The following describes the details of acquisition of a detection signal resulting from the acoustic wave AW and generation of a photoacoustic image based on the acquired detection signal by the photoacoustic imaging apparatus 100 by referring to FIG. 4. The following description is about acquisition of a detection signal resulting from the acoustic wave AW and generation of a photoacoustic image. Thus, a signal simply called a detection signal means a detection signal resulting from the acoustic wave AW.

As shown in FIG. 4, in the photoacoustic imaging apparatus 100, the control portion 30 is configured to acquire a detection signal at a sampling frequency that determines a sampling cycle SC to be one cycle.

In the first embodiment, the control portion 30 is configured to make the light source portion 10 emit pulsed light and to acquire a detection signal resulting from the acoustic wave AW in the sampling cycle SC shorter than the refresh cycle RC. The control portion 30 is also configured to generate a photoacoustic image (photoacoustic image data) by executing averaging processing by taking a moving average using the detection signal detected in the sampling cycle SC.

More specifically, the control portion 30 is configured to make the light source portion 10 emit pulsed light and to acquire a detection signal in a detecting section belonging to the sampling cycle SC and shown schematically by a substantially rectangular pulse wave.

The control portion 30 is configured to execute the following in a signal processing section belonging to the sampling cycle SC and following the detecting section of the same sampling cycle SC: storing of a detection signal acquired in this sampling cycle SC into the storage portion 30a, averaging processing by taking of a moving average using a plurality of stored detection signals, etc. The control portion 30 is also configured to generate photoacoustic image data by executing averaging processing by taking a moving average, to output the generated photoacoustic image data to the display portion 40, and to make the display portion 40 display a photoacoustic image.

In the first embodiment, the sampling cycle SC has a length shorter than that of the refresh cycle RC and allowing the light source portion 10 to emit pulsed light several times while allowing a detection signal to be acquired several times within the refresh cycle RC. Specifically, in the first embodiment, several detecting sections exist within one refresh cycle RC, as shown in FIG. 4.

The length of the sampling cycle SC is preferably determined in consideration of an MPE (maximum permission exposure) of skin. The reason for this is that decrease in the length of the sampling cycle SC reduces an MPE value. If a measurement wavelength is 750 nm, the pulse width of pulsed light is 1 microsecond, and the sampling cycle SC is 0.1 msec, for example, an MPE value of skin is about 14 J/m². If the peak power of pulsed light emitted from the light source portion 10 is 3 kW and an area of emission from the light source portion 10 is 250 mm², for example, the energy of light emitted from the light source portion 10 to the test object P such as a human body is about 12 J/m². Thus, the energy of light emitted from the light source portion 10 described in this example does not exceed the MPE value, so that the sampling cycle SC can be set at 0.1 msec or more. Specifically, the sampling cycle SC is preferably set in a range not to cause excess over the MPE value.

As shown in FIG. 4, in the first embodiment, the control portion 30 is configured to generate a photoacoustic image (photoacoustic image data) to be displayed on the display portion 40 by taking a moving average of detection signals during averaging processing by taking of a moving average, with the number of additions (in FIG. 4, five) that makes a value (time) obtained by multiplying the time of the sampling cycle SC and the number of additions during taking of a moving average exceed the time of the refresh cycle RC. If the refresh cycle RC is 16.7 msec and the sampling cycle SC is 0.1 msec, for example, the number of additions satisfying this requirement is 168 or more.

The control portion 30 is configured to generate a photoacoustic image (photoacoustic image data) to be displayed on the display portion 40 by taking a moving average of detection signals with the number of additions that makes a value (time) obtained by multiplying the time of the sampling cycle SC and the number of additions during taking of a moving average about 125 msec or less. If the sampling cycle SC is 0.1 msec, for example, the number of additions satisfying this requirement is 1250 or less. Thus, if the refresh cycle RC is 16.7 msec and the sampling cycle SC is 0.1 msec, for example, the number of additions satisfying the aforementioned requirements is set in a range from 168 to 1250.

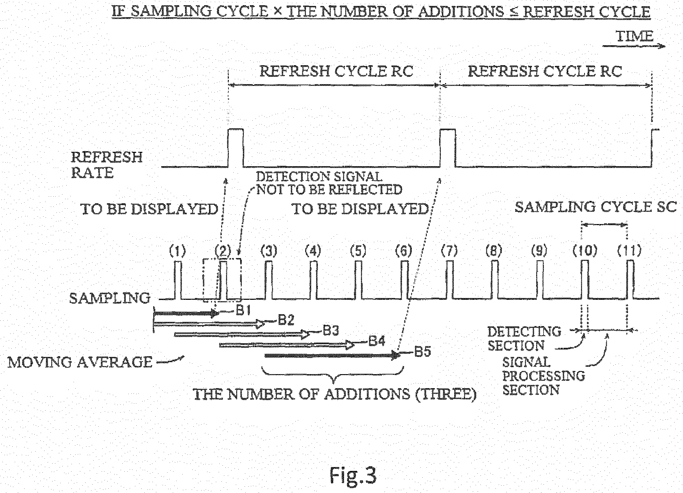

Averaging processing by taking of a moving average and display of a generated photoacoustic image will be described next by referring to FIGS. 3 and 4 and giving specific examples. In a case described first by referring to FIG. 3, a value (time) obtained by multiplying the time of the sampling cycle SC and the number of additions during taking of a moving average does not exceed the time of the refresh cycle RC (sampling cycle SC × the number of additions ≤ refresh cycle RC). Conditions such as numerical values given in the following description are illustrative and not restrictive.

FIG. 3 shows averaging processing by taking of a moving average schematically using an arrow B1, an arrow B2, an arrow B3, an arrow B4, and an arrow B5. Specifically, the arrows B1 to B5 show that averaging processing by taking of a moving average is executed on every occasion (each time a detection signal is acquired), with the number of additions set at three. For example, the arrow B4 shows that averaging processing is being executed by taking a moving average of detection signals acquired in a detecting section (2), a detecting section (3), and a detecting section (4) respectively. The arrow B5 shows that averaging processing is being executed by taking a moving average of detection signals acquired in the detecting section (3), the detecting section (4), and a detecting section (5) shifted subsequently by one detecting section from those shown by the arrow B4. Averaging processing executed by taking a moving average is also executed before the arrow B1 and after the arrow B5. However, for the sake of simplicity, such averaging processing is omitted from the drawing.

In the photoacoustic imaging apparatus 100, an image displayed on the screen 40a of the display portion 40 (see FIG. 1) is a photoacoustic image generated by averaging processing (a photoacoustic image already generated) to coincide with timing of refresh (update) of the screen 40a in the refresh cycle RC. Specifically, in FIG. 3, images displayed on the screen 40a of the display portion 40 are photoacoustic images generated by averaging processing corresponding to the arrow B1 shown as a black arrow and averaging processing corresponding to the arrow B5 shown as a black arrow. Specifically, the photoacoustic image generated by the averaging processing corresponding to the arrow B1 is displayed. Then, the photoacoustic image generated by the averaging processing corresponding to the arrow B5 is displayed as a next photoacoustic image.

If sampling cycle SC × the number of additions ≤ refresh cycle RC, while detection signals are acquired in the detecting sections (2) to (5) in a period from the arrow B1 to the arrow B5, a detection signal to contribute to a photoacoustic image includes only the detection signals corresponding to the detecting sections (3), (4), and (5) covered by the arrow B5 out of those corresponding to the detecting sections (2) to (5). This unfortunately generates a detection signal such as one corresponding to the detecting section (2) not to be reflected in generation of a photoacoustic image.

By contrast, unlike the case of FIG. 3, in the case of FIG, 4, the photoacoustic imaging apparatus 100 according to the first embodiment does not generate a detection signal not to be reflected in generation of a photoacoustic image. In a case described next by referring to FIG. 4, a value (time) obtained by multiplying the time of the sampling cycle SC and the number of additions during taking of a moving average exceeds the time of the refresh cycle RC (sampling cycle SC × the number of additions > refresh cycle RC).

FIG. 4 shows averaging processing by taking of a moving average schematically using an arrow B11, an arrow B12, an arrow B13, an arrow B14, an arrow B15, and an arrow B16. Specifically, the arrows B11 to B16 show that averaging processing by taking of a moving average is executed on every occasion (each time a detection signal is acquired), with the number of additions set at five. Averaging processing by taking of a moving average is also executed before the arrow B11 and after the arrow B16. However, for the sake of simplicity, such averaging processing is omitted from the drawing.

In FIG. 4, averaging processing corresponding to the arrow B11 shown as a black arrow and averaging processing corresponding to the arrow B 16 shown as a black arrow are executed to coincide with timing of refresh (update) of the screen 40a in the refresh cycle RC. Thus, images displayed on the screen 40a of the display portion 40 are photoacoustic images generated by the averaging processing corresponding to the arrow B11 shown as the black arrow and the averaging processing corresponding to the arrow B16 shown as the black arrow. Specifically, the photoacoustic image generated by the averaging processing corresponding to the arrow B11 is displayed. Then, the photoacoustic image generated by the averaging processing corresponding to the arrow B16 is displayed as a next photoacoustic image.

In the case of the photoacoustic imaging apparatus 100 according to the first embodiment where sampling cycle SC × the number of additions > refresh cycle RC, the arrow B16 covers all the detection signals corresponding to the detecting sections (6) to (10) acquired in a period from the arrow B11 to the arrow B16. Thus, there will be no detection signal not to be reflected in generation of a photoacoustic image. For the convenience of illustration, the arrows B11 and B16 have been described as examples. However, detection signals acquired before the arrow B11 and detection signals acquired after the arrow B16 also do not include a detection signal not to be reflected in generation of a photoacoustic image. As described above, in the photoacoustic imaging apparatus 100 according to the first embodiment, a value (time) obtained by multiplying the time of the sampling cycle SC and the number of additions during taking of a moving average is set to exceed the time of the refresh cycle RC, thereby reducing the likelihood of the occurrence of a detection signal not to be reflected in generation of a photoacoustic image.

The first embodiment achieves the following effect.

As described above, in the first embodiment, the control portion 30 is provided that makes the semiconductor light-emitting element light source portion 10 emit light and acquires a detection signal in the sampling cycle SC shorter than the refresh cycle RC. Further, the control portion 30 generates a photoacoustic image to be displayed on the display portion 40 using the detection signal detected in the sampling cycle SC. As the sampling cycle SC is set to be shorter than the refresh cycle RC, a detection signal can be acquired within the refresh cycle RC. Thus, the detection signal acquired within the refresh cycle RC can be used for generating an image. This can easily reduce the likelihood of failing to update the screen 40a of the display portion 40 (displaying one image continuously) due to failing to acquire a detection signal during a cycle of updating the screen 40a (refresh cycle RC). This can easily make it less likely that the cycle of updating the screen 40a will be substantially extended. As a result, awkward motion of moving images displayed on the screen 40a (loss of smoothness) can be suppressed. Further, provision of the semiconductor light-emitting element light source portion 10 can shorten the sampling cycle SC easily, compared to use of a solid-laser light source portion having difficultly in shortening a light emission cycle (sampling cycle SC).

As described above, in the first embodiment, the control portion 30 is configured to generate a photoacoustic image to be displayed on the display portion 40 by taking a moving average of detection signals detected in the sampling cycles SC. By doing so, a moving average of the detection signals detected in the sampling cycles SC is taken, so that a photoacoustic image can be generated with an increased S/N ratio (signal-to-noise ratio).

As described above, in the first embodiment, the sampling cycle SC has a length that allows the semiconductor light-emitting element light source portion 10 to emit light several times while allowing a detection signal to be acquired several times within the refresh cycle RC. Thus, the several detection signals can be acquired within the refresh cycle RC. This can more easily reduce the likelihood of failing to update the screen 40a of the display portion 40 due to failing to acquire a detection signal during a cycle of updating the screen 40a (refresh cycle RC). As a result, awkward motion of moving images displayed on the screen 40a can be suppressed more easily.

As described above, in the first embodiment, the control portion 30 is configured to generate a photoacoustic image to be displayed on the display portion 40 by taking a moving average of detection signals with the number of additions that makes a value obtained by multiplying the sampling cycle SC and the number of additions during taking of a moving average exceed the refresh cycle RC. This can reduce the likelihood of the occurrence of a detection signal out of acquired detection signals and not to be reflected in generation of a photoacoustic image as shown in FIG. 4, compared to the case of FIG. 3 where a value obtained by multiplying the sampling cycle SC and the number of additions during taking of a moving average does not exceed the refresh cycle RC.

As described above, in the first embodiment, the control portion 30 is configured to generate a photoacoustic image to be displayed on the display portion 40 by taking a moving average as an arithmetic average. This makes it possible to generate a photoacoustic image in such a manner as to smoothen a motion (difference) between individual generated photoacoustic images, unlike the case of taking a average as an arithmetic average. Thus, moving images formed of the individual photoacoustic images can be displayed smoothly. This configuration works effectively, particularly in displaying moving images of the inside of the test object P to change constantly such as a human body.

As described above, in the first embodiment, the control portion 30 is configured to take a moving average each time a detection signal is acquired. By doing so, averaging processing is executed on every occasion on each detection signal by taking a moving average of detection signals, so that averaging processing for generating a photoacoustic image can be executed reliably at each refresh rate. As a result, a photoacoustic image can be generated reliably during update of the screen 40a, making it possible to reliably reduce the likelihood of failing to update the screen 40a. Thus, awkward motion of moving images displayed on the screen 40a can be suppressed reliably.

As described above, in the first embodiment, the control portion 30 is configured to generate a photoacoustic image to be displayed on the display portion 40 by taking an arithmetic average of detection signals with the number of additions that makes a value obtained by multiplying the sampling cycle SC and the number of additions during taking of an arithmetic average about 125 msec or less. Thus, the redundancy of averaging time can be suppressed. This can suppress awkward motion of moving images formed of individual photoacoustic images due to redundancy of averaging time.

As described above, in the first embodiment, the semiconductor light-emitting element 11b includes at least one of a light-emitting diode element, a semiconductor laser element, and an organic light-emitting diode element. If the light-emitting diode element is used as the semiconductor light-emitting element 11b, using the light-emitting diode element of relatively low power consumption can reduce power consumption. If the semiconductor laser element is used as the semiconductor light-emitting element 11b, a laser beam of relatively high directivity can be emitted to the test object. Thus, much of the beam from the semiconductor laser element can be applied reliably to the test object P. If the organic light-emitting diode element is used as the semiconductor light-emitting element 11b, using the organic light-emitting diode element capable of being reduced in thickness easily facilitates size reduction of the semiconductor light-emitting element light source portion 10 to include the organic light-emitting diode element.

### (Second Embodiment)

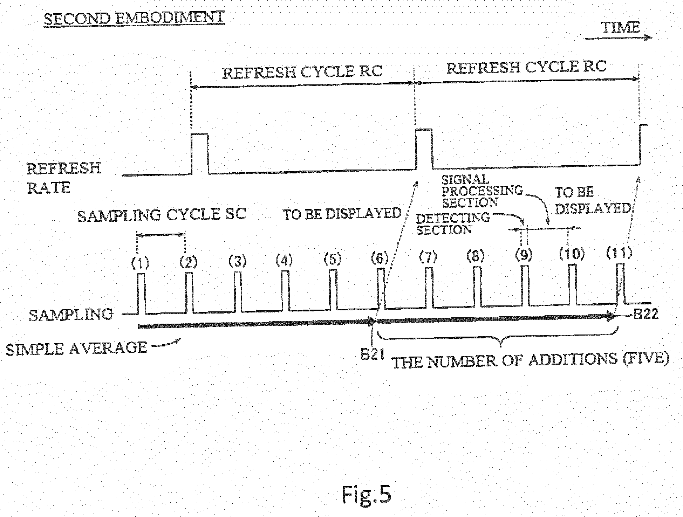

A second embodiment will be described next by referring to FIGS. 1 and 5. In an example described in the second embodiment, unlike the aforementioned configuration of the first embodiment where averaging processing is executed by taking a moving average, averaging processing is executed by taking a average. Structures comparable to those of the first embodiment will be given the same signs and description of such structures will be omitted.

As shown in FIG 1, a photoacoustic imaging apparatus 200 according to the second embodiment of the present invention includes a control portion 130.

As shown in FIG 5, like in the first embodiment, the sampling cycle SC has a length shorter than that of the refresh cycle RC and allowing the semiconductor light-emitting element light source portion 10 (hereinafter called the "light source portion 10") to emit light several times while allowing a detection signal to be acquired several times within the refresh cycle RC. In the second embodiment, the control portion 130 is configured to make the light source portion 10 emit pulsed light and to acquire a detection signal resulting from the acoustic wave AW in the sampling cycle SC shorter than the refresh cycle RC. The control portion 130 is also configured to generate a photoacoustic image (photoacoustic image data) by executing averaging processing by taking a average using a detection signal detected in the sampling cycle SC.

The control portion 130 is configured to generate a photoacoustic image (photoacoustic image data) to be displayed on the display portion 40 by taking a average of detection signals during averaging processing by taking of a average, with the number of additions (in FIG. 5, five) that makes a value (time) obtained by multiplying the time of the sampling cycle SC and the number of additions during taking of a average exceed the time of the refresh cycle RC.

Averaging processing by taking of a average and display of a generated photoacoustic image will be described next by referring to FIG. 5 using specific examples. Conditions such as numerical values given in the following description are illustrative and not restrictive.

FIG. 5 shows averaging processing by taking of a average schematically using an arrow B21 and an arrow B22. Specifically, the arrows B21 and B22 show that averaging processing by taking of a average is executed in units of detection signals of a number corresponding to the number of additions. For example, the arrow B21 shows that averaging processing is being executed by taking a average of detection signals acquired in a detecting section (1), a detecting section (2), a detecting section (3), a detecting section (4), and a detecting section (5) respectively. The arrow B22 shows that averaging processing is being executed by taking a average of detection signals acquired in a detecting section (6), a detecting section (7), a detecting section (8), a detecting section (9), and a detecting section (10) respectively after the arrow B21. Averaging processing by taking of a average is also executed before the arrow B21 and after the arrow B22.

In the photoacoustic imaging apparatus 200, an image displayed on the screen 40a of the display portion 40 is a photoacoustic image generated by averaging processing (a photoacoustic image already generated) to coincide with timing of refresh (update) of the screen 40a in the refresh cycle RC. Specifically, in FIG. 5, images displayed on the display portion 40 are photoacoustic images generated by averaging processing corresponding to the arrow B21 shown as a black arrow and averaging processing corresponding to the arrow B22 shown as a black arrow.

Thus, in the case of the photoacoustic imaging apparatus 200 according to the second embodiment, the arrow B22 also covers all the detection signals corresponding to the detecting sections (6) to (10) acquired in a period from the arrow B21 to the arrow B22. Thus, there will be no detection signal not to be reflected in generation of a photoacoustic image. For the convenience of illustration, the arrows B21 and B22 have been described as examples. However, detection signals acquired before the arrow B21 and detection signals acquired after the arrow B22 also do not include a detection signal not to be reflected in generation of a photoacoustic image.

The configuration of the second embodiment is the same in the other respects as that of the first embodiment.

The second embodiment achieves the following effect.

As described above, in the second embodiment, the control portion 130 is provided that makes the semiconductor light-emitting element light source portion 10 emit light and acquires a detection signal in the sampling cycle SC shorter than the refresh cycle RC. Further, the control portion 130 generates a photoacoustic image to be displayed on the display portion 40 by taking a average of detection signals detected in the sampling cycles SC. As a result, like in the first embodiment, awkward motion of moving images displayed on the screen 40a (loss of smoothness) can be suppressed in the second embodiment.

As described above, in the second embodiment, the control portion 130 is configured to generate a photoacoustic image to be displayed on the display portion 40 by taking a average as an arithmetic average. This makes it possible to reduce the number of detection signals to be stored (recorded) in the storage portion 30a (memory), compared to taking a moving average as an arithmetic average. This achieves a corresponding saving of the volume of the storage portion 30a (memory).

The other effect of the second embodiment is the same as that achieved by the first embodiment.

### (Third Embodiment)

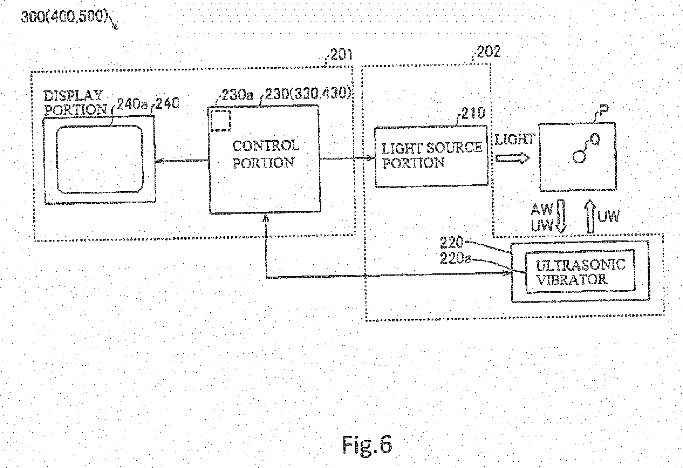

A third embodiment will be described next by referring to FIGS. 6 to 8. In an example described in the third embodiment, a average signal is generated using a detection signal and an image to be displayed on a display portion is generated based on the generated average signal.

The configuration of a photoacoustic imaging apparatus 300 according to the third embodiment of the present invention will be described by referring to FIGS. 6 to 8.

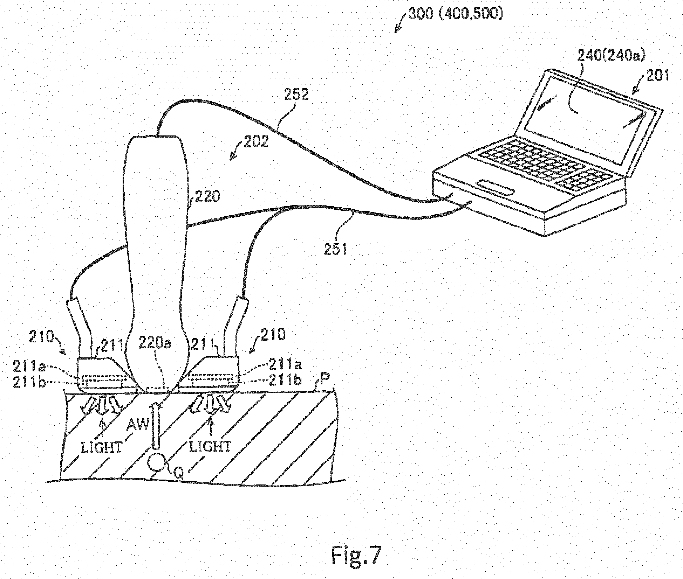

As shown in FIGS. 6 and 7, the photoacoustic imaging apparatus 300 according to the third embodiment of the present invention includes an apparatus body 201 and a probe portion 202. The apparatus body 201 and the probe portion 202 are connected through a wire 251 and a wire 252. The apparatus body 201 includes a control portion 230 and a display portion 240. The probe portion 202 includes a light source portion 210 and a detecting portion 220. The light source portion 210 is an example of a "semiconductor light-emitting element light source portion" according to the present invention.

As shown in FIGS. 6 and 7, the light source portion 210 includes two light sources 211 and is configured to emit pulsed light for measurement from each of the two light sources 211 toward a test object P. Each of the two light sources 211 is connected through the wire 251 to the apparatus body 201 and is configured to receive power and a control signal supplied from the apparatus body 201 through the wire 251.

The two light sources 211 are arranged near the detecting portion 220 and on opposite sides of the detecting portion 220 so as to sandwich the detecting portion 220 therebetween. In this way, the two light sources 211 are configured to emit pulsed light toward the test object P from different positions.

Each of the two light sources 211 includes a light source substrate 211 a and a semiconductor light-emitting element 211b. For example, a light-emitting diode element, a semiconductor laser element, or an organic light-emitting diode element is applicable as the semiconductor light-emitting element 211b. The light source substrate 211a has a lower surface holding a plurality of semiconductor light-emitting elements 211b mounted in an array pattern. The light source substrate 211 a is configured to make the semiconductor light-emitting element 211b emit a pulse based on a control signal output from the control portion 230.

The two semiconductor light-emitting elements 211b are both configured to generate light of a measurement wavelength in an infrared region suitable for measurement of the test object P such as a human body (light having a center wavelength from about 700 to about 1000 nm, for example). The two semiconductor light-emitting elements 211b may be configured to generate light of different measurement wavelengths or light of the substantially same measurement wavelength. A measurement wavelength may be determined properly in a manner that depends on an intended detection target Q to be detected.

As shown in FIGS. 6 and 7, the detecting portion 220 is an ultrasonic probe and connected to the apparatus body 201 through the wire 252. The detecting portion 220 includes an ultrasonic vibrator 220a. In the detecting portion 220, the ultrasonic vibrator 220a includes a plurality of ultrasonic vibrators 220a arranged in an array pattern. The detecting portion 220 is configured to detect an acoustic wave (ultrasonic wave) AW in response to vibration of the ultrasonic vibrator 220a caused by an acoustic wave generated from the detection target Q in the test object P when the detection target Q absorbs pulsed light emitted from the light source portion 210. The detecting portion 220 is configured to be capable of generating an ultrasonic wave UW by making the ultrasonic vibrator 220a vibrate based on a control signal output from the control portion 230. The detecting portion 220 is also configured to detect the ultrasonic wave UW in response to vibration of the ultrasonic vibrator 220a caused by this ultrasonic wave UW reflected in the test object P. The detecting portion 220 is configured to output a detection signal corresponding to the detected acoustic wave AW or the detected ultrasonic wave UW to the control portion 230 through the wire 252.

In this description, for the convenience of explanation, an ultrasonic wave generated by absorption of pulsed light by the detection target Q in the test object P will be called an "acoustic wave," an ultrasonic wave generated by the ultrasonic vibrator 220a and reflected in the test object P will be called an "ultrasonic wave," and the "acoustic wave" and the "ultrasonic wave" will be described distinctively.

The control portion 230 includes a CPU and a storage portion 230a such as a ROM or a RAM. As shown in FIG 6, the control portion 230 is configured to form an image of the inside of the test object P based on a detection signal output from the detecting portion 220. More specifically, the control portion 230 is configured to generate a photoacoustic image based on a detection signal resulting from the acoustic wave AW and to generate an ultrasonic image based on a detection signal resulting from the ultrasonic wave UW. The control portion 230 is configured to be capable of forming an image of various types of information in the test object P by synthesizing the photoacoustic image and the ultrasonic image. Generation of the photoacoustic image resulting from the acoustic wave AW will be described in detail later.

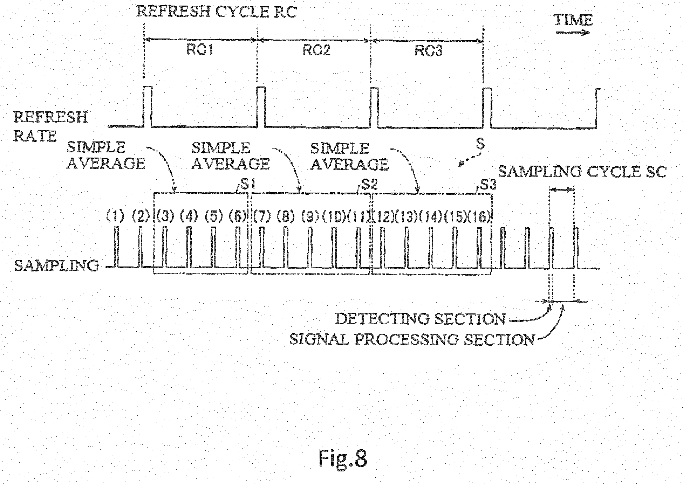

As shown in FIG. 6, the display portion 240 is formed of a general liquid crystal type monitor or a scanning type monitor. The display portion 240 has a screen 240a and is configured to display an image such as an image of the inside of the test object P on the screen 240a formed by the control portion 230. As shown in FIG. 8, the display portion 240 is configured in such a manner that the screen 240a is updated at a certain refresh rate (frequency). The certain refresh rate to be applied is generally about 50 Hz or more (about 50 Hz, about 60 Hz, or about 120 Hz, for example), and these values are applicable as the certain refresh rate.

The refresh rate mentioned herein is a value indicating the number of times the screen 240a is refreshed (updated) per unit time (one second, for example). This means that, if the refresh rate is about 60 Hz, the screen 240a is refreshed about 60 times in one second. In other words, this means that the screen 240a is refreshed in each cycle of a refresh rate (hereinafter called a refresh cycle) RC of about 16.7 msec. In FIG. 8, timing of refresh of the screen 240a is schematically shown by a substantially rectangular pulse wave. In the case of a liquid crystal type monitor, for example, the screen 240a is refreshed at the time of rising of a substantially rectangular pulse wave. In the case of a scanning type monitor, for example, the monitor is scanned within the refresh cycle RC to refresh the screen 240a.

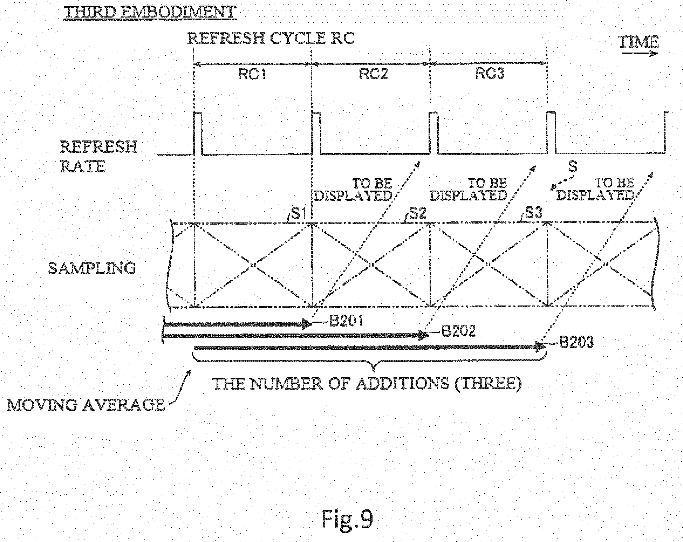

The following describes the details of generation of a photoacoustic image by the photoacoustic imaging apparatus 300 according to the third embodiment by referring to FIGS. 8 and 9. The following description is about acquisition of a detection signal resulting from the acoustic wave AW and generation of a photoacoustic image. Thus, a signal simply called a detection signal means a detection signal resulting from the acoustic wave AW.

In outline, the photoacoustic imaging apparatus 300 acquires detection signals resulting from the acoustic waves AW and executes averaging processing on the acquired detection signals. Then, the photoacoustic imaging apparatus 300 generates a photoacoustic image based on the detection signals having been subjected to the averaging processing. This averaging processing is intended to increase an S/N ratio by executing averaging processing on individual detection signals. The following describes these processing steps sequentially.

As shown in FIG. 8, in the photoacoustic imaging apparatus 300, the control portion 230 is configured to acquire a detection signal at a sampling frequency (any frequency from 1 to 10 kHz, for example) that determines a certain sampling cycle (hereinafter simply called a sampling cycle) SC to be one cycle.

More specifically, the control portion 230 is configured to make the light source portion 210 emit pulsed light and to acquire a detection signal in a detecting section belonging to the sampling cycle SC and shown by a substantially rectangular pulse wave.

The control portion 230 is configured to execute the following in a signal processing section belonging to the sampling cycle SC and following the detecting section of the same sampling cycle SC: storing of a detection signal acquired in this sampling cycle SC into the storage portion 230a, averaging processing by using a plurality of stored detection signals, etc.

The sampling cycle SC has a length shorter than that of the refresh cycle RC and allowing the light source portion 210 to emit pulsed light several times while allowing a detection signal to be acquired several times within the refresh cycle RC.

As shown in FIG. 8, in the third embodiment, the control portion 230 is configured to generate a average signal S (S1, S2, and S3 shown in FIG. 8) by taking a average of a plurality of detection signals from a detection signal acquired first to a detection signal acquired last within one refresh cycle RC. FIG. 8 schematically shows the average signal S (S1 to S3) by surrounding detection signals by chain double-dashed lines that are used for a average. The detection signal to be acquired first can be a detection signal acquired immediately after timing of one refresh (at the time of rising of a substantially rectangular pulse wave), for example. The detection signal to be acquired last can be a detection signal acquired immediately before timing of refresh subsequent to the timing of the former refresh.

As shown in FIG. 9, in the third embodiment, the control portion 230 is configured to generate a photoacoustic image (photoacoustic image data) by defining the average signal S as one unit and acquiring the average signals S as a plurality of units (in FIG. 9, thee units), and by executing averaging processing further by taking a moving average using the acquired average signals S as the plurality of units. The control portion 230 is configured to display the photoacoustic image on the screen 240a of the display portion 240 by outputting the generated photoacoustic image data to the display portion 240.

In the third embodiment, the control portion 230 is configured to acquire the average signals S as a plurality of units in such a manner that the number of detection signals to be subjected to averaging processing (in other words, the number of detecting sections shown in FIG. 8) reaches a certain number or more during averaging processing by taking of a moving average of the average signals S. If the certain number is 100 and one average signal S includes 20 detection signals on average, for example, average signal S as five units or more are acquired. The certain number is determined properly by experiment conducted in consideration of an S/N ratio desirable for imaging, for example.

The control portion 230 is configured to acquire the average signals S as a plurality of units in such a manner that a value (averaging time) obtained by multiplying the time of the sampling cycle SC and the number of detection signals to be subjected to averaging processing is about 125 msec or less. If the sampling cycle SC is about 1 msec, for example, the number of detection signals to be subjected to averaging processing is 125 (125 times) or less. Thus, if the average signal S includes 20 detection signals on average, the average signals S of less than seven units are acquired. By doing so, the redundancy of averaging time can be suppressed. This can suppress awkward motion of moving images formed of individual photoacoustic images due to redundancy of averaging time.

The aforementioned processing will be described next by referring to FIGS. 8 and 9 and giving specific examples. Conditions such as numerical values given in the following description are illustrative and not restrictive.

FIG. 8 shows generation of the average signal S by taking a average of detection signals within the refresh cycle RC. FIG. 8 shows three average signals S1 to S3 acquired as the average signal S.

The average signal S1 is a signal generated by taking a average of four detection signals from a detection signal acquired first in a detecting section (3) within a refresh cycle RC1 to a detection signal acquired last in a detecting section (6) within the refresh cycle RC1. Likewise, the average signal S2 is a signal generated by taking a average of five detection signals from a detection signal acquired first in a detecting section (7) within a refresh cycle RC2 to a detection signal acquired last in a detecting section (11) within the refresh cycle RC2. Likewise, the average signal S3 is a signal generated by taking a average of five detection signals from a detection signal acquired first in a detecting section (12) within a refresh cycle RC3 to a detection signal acquired last in a detecting section (16) within the refresh cycle RC3. The average signal S is also generated before generation of the average signal S1 and after generation of the average signal S3. However, for the sake of simplicity, generation of such average signals is omitted from the drawing.

FIG. 9 shows generation of a photoacoustic image by executing averaging processing further by taking a moving average using the average signals S as a plurality of units. FIG. 9 shows averaging processing by taking of a moving average schematically using an arrow B201, an arrow B202, and an arrow B203. Specifically, the arrows B201 to B203 show that averaging processing on the average signals S is executed by taking a moving average on every occasion (in units of average signals S), with the number of additions set at three. For example, the arrow B203 shows that averaging processing is being executed by taking a moving average of the average signals S1 to S3.

In the photoacoustic imaging apparatus 300, an image displayed on the screen 240a of the display portion 240 is a photoacoustic image generated by averaging processing (a photoacoustic image already generated) to coincide with timing of refresh (update) of the screen 240a in the refresh cycle RC. As described above, the photoacoustic imaging apparatus 300 generates a photoacoustic image by executing averaging processing while defining the average signal S as one unit, so that all acquired detection signals can be used for generating the photoacoustic image.

The third embodiment achieves the following effect.

As described above, in the third embodiment, the control portion 230 is provided that generates the average signal S by taking a average of detection signals from a detection signal acquired first within the refresh cycle RC to a detection signal acquired last within this refresh cycle RC. The control portion 230 generates a photoacoustic image to be displayed on the display portion 240 based on the average signal S. By doing so, all detection signals acquired within the refresh cycle RC can be used for generating a photoacoustic image. This can reduce the likelihood of the occurrence of a detection signal out of acquired detection signals and not to be reflected in a photoacoustic image to be displayed on the screen 240a of the display portion 240. As a result, the acquired detection signals can be used efficiently for generating a photoacoustic image.

As described above, in the third embodiment, the control portion 230 is configured to generate a photoacoustic image to be displayed on the display portion 240 by defining the average signal S generated by taking a average as one unit and acquiring the average signals S as a plurality of units (three units), and by taking an arithmetic average of the acquired average signals S as the plurality of units. This achieves generation of a photoacoustic image by executing averaging processing on more detection signals, so that the photoacoustic image can be generated with an increased S/N ratio (signal-to-noise ratio). As a result, a clear photoacoustic image can be generated through efficient use of acquired detection signals.

As described above, in the third embodiment, the control portion 230 is configured to generate a photoacoustic image to be displayed on the display portion 240 by taking a moving average as an arithmetic average for taking an arithmetic average of the average signals S. By doing so, while the average signals S are generated by taking a average, a moving average of these average signals S is taken. This makes it possible to generate a photoacoustic image in such a manner as to smoothen a motion (difference) between individual photoacoustic images. Thus, moving images formed of the individual photoacoustic images can be displayed smoothly while acquired detection signals are used efficiently. This configuration of taking a moving average works effectively, particularly in displaying moving images of the inside of the test object P to change constantly such as a human body.

As described above, in the third embodiment, the control portion 230 is configured to take a moving average each time the average signal S is acquired. By doing so, averaging processing is executed on every occasion on each average signal S by taking a moving average of the average signals S, so that averaging processing for generating a photoacoustic image can be executed reliably at each refresh rate. As a result, a photoacoustic image can be generated reliably during update of the screen 240a, making it possible to reliably reduce the likelihood of failing to update the screen 240a. In this way, while an image is generated by efficiently using acquired detection signals, awkward motion of moving images displayed on the screen 240a can be suppressed reliably.

As described above, in the third embodiment, the control portion 230 is configured to acquire the average signals S as a plurality of units in such a manner that a value obtained by multiplying the sampling cycle SC and the number of detection signals to be subjected to averaging processing by taking of an arithmetic average is about 125 msec or less. By doing so, the redundancy of averaging time can be suppressed. This can suppress awkward motion of moving images formed of individual photoacoustic images due to redundancy of averaging time.

As described above, in the third embodiment, the control portion 230 is configured to define the average signal S generated by taking a average as one unit and acquire the average signals S as a plurality of units in such a manner that the number of detection signals reaches a certain number or more. The control portion 230 is also configured to generate a photoacoustic image to be displayed on the display portion 240 based on the acquired average signals S as the plurality of units. This makes it possible to execute averaging processing reliably using detection signals of the certain number or more, so that an S/N ratio (signal-to-noise ratio) can be increased reliably during generation of a photoacoustic image. As a result, a clear photoacoustic image can be generated reliably.

As described above, in the third embodiment, the semiconductor light-emitting element 211b includes at least one of a light-emitting diode element, a semiconductor laser element, and an organic light-emitting diode element. If the light-emitting diode element is used as the semiconductor light-emitting element 211 b, using the light-emitting diode element of relatively low power consumption can reduce power consumption. If the semiconductor laser element is used as the semiconductor light-emitting element 211b, a laser beam of relatively high directivity can be emitted to the test object. Thus, much of the beam from the semiconductor laser element can be applied reliably to the test object P. If the organic light-emitting diode element is used as the semiconductor light-emitting element 211b, using the organic light-emitting diode element capable of being reduced in thickness easily facilitates size reduction of the light source portion 210 to include the organic light-emitting diode element.

### (Fourth Embodiment)

A fourth embodiment will be described next by referring to FIGS. 6 to 8 and 10. In an example described in the fourth embodiment, unlike the aforementioned configuration of the third embodiment where averaging processing on the average signals S is executed by taking a moving average, averaging processing on the average signals S is executed by taking a average. Structures comparable to those of the third embodiment will be given the same signs and description of such structures will be omitted.

As shown in FIG 6, a photoacoustic imaging apparatus 400 according to the fourth embodiment of the present invention (see FIG. 7) includes a control portion 330.

As shown in FIG. 8, like in the third embodiment, the control portion 330 is configured to generate the average signal S (S1, S2, and S3 shown in FIG. 8) by taking a average of a plurality of detection signals from a detection signal acquired first to a detection signal acquired last within one refresh cycle RC.

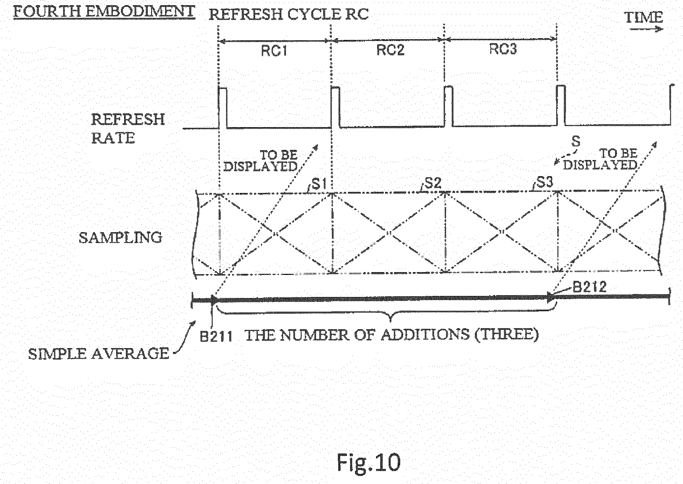

As shown in FIG. 10, in the fourth embodiment, the control portion 330 is configured to generate a photoacoustic image (photoacoustic image data) by defining the average signal S as one unit and acquiring the average signals S as a plurality of units (in FIG. 10, three units), and by executing averaging processing further by taking a average using the acquired average signals S as the plurality of units. The control portion 330 is configured to display the photoacoustic image on the screen 240a of the display portion 240 by outputting the generated photoacoustic image data to the display portion 240.

The aforementioned processing will be described next by referring to FIG. 10 and giving specific examples. Conditions such as numerical values given in the following description are illustrative and not restrictive. Processing of generating the average signal S shown in FIG. 8 is the same as the above-described processing of the third embodiment, so that description of this processing will be omitted.

FIG. 10 shows generation of a photoacoustic image by executing averaging processing further by taking a average using the average signals S as a plurality of units. FIG. 10 shows averaging processing by taking of a average schematically using an arrow B211 and an arrow B212. Specifically, the arrows B211 and B212 show that averaging processing on the average signals S is executed by taking a average in units of average signals S of a number corresponding to the number of additions, with the number of additions set at three. For example, the arrow B212 shows that averaging processing is being executed by taking a average of the average signals S1 to S3.

In the photoacoustic imaging apparatus 400, an image displayed on the screen 240a of the display portion 240 is also a photoacoustic image generated by averaging processing (a photoacoustic image already generated) to coincide with timing of refresh (update) of the screen 240a in the refresh cycle RC. As described above, the photoacoustic imaging apparatus 400 also generates a photoacoustic image by executing averaging processing while defining the average signal S as one unit, so that all acquired detection signals can also be used for generating the photoacoustic image.

The configuration of the fourth embodiment is the same in the other respects as that of the third embodiment.

The fourth embodiment achieves the following effect.

As described above, in the fourth embodiment, the control portion 330 is provided that generates a photoacoustic image to be displayed on the display portion 240 based on the average signal S. By doing so, in the fourth embodiment, all acquired detection signals can be used efficiently for generating a photoacoustic image, like in the third embodiment.

As described above, in the fourth embodiment, the control portion 330 is configured to generate a photoacoustic image to be displayed on the display portion 240 by taking a average as an arithmetic average for taking an arithmetic average of the average signals S. This makes it possible to reduce the number of detection signals to be stored (recorded) in the storage portion 230a (memory), compared to taking a moving average as an arithmetic average. This achieves a corresponding saving of the volume of the storage portion 230a (memory).

The other effect of the fourth embodiment is the same as that achieved by the third embodiment.

### (Fifth Embodiment)

A fifth embodiment will be described next by referring to FIGS. 6, 7, and 11. In an example described in the fifth embodiment, unlike the aforementioned configuration of the third embodiment, averaging processing is executed while the refresh cycle RC and the sampling cycle SC are synchronized with each other. Structures comparable to those of the third embodiment will be given the same signs and description of such structures will be omitted.

As shown in FIG. 6, a photoacoustic imaging apparatus 500 according to the fifth embodiment of the present invention (see FIG. 7) includes a control portion 430.

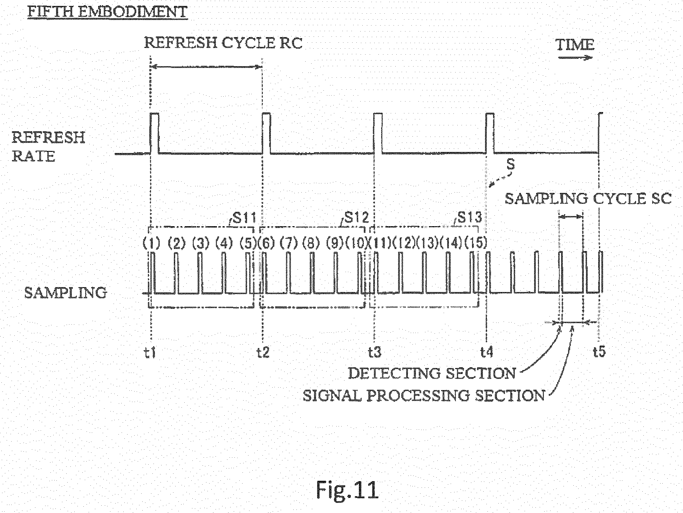

As shown in FIG. 11, in the fifth embodiment, the control portion 430 is configured to synchronize timing of start of the refresh cycle RC and timing of start of the sampling cycle SC with each other for each refresh cycle RC. More specifically, in FIG. 11, timing of start of the refresh cycle RC and timing of start of the sampling cycle SC are synchronized with each other at a moment t1, a moment t2, a moment t3 a moment t4, a moment t5, and at subsequent moments. In this case, as shown in FIG. 11, only the last sampling cycle SC within the refresh cycle RC is changed in length from the other sampling cycles SC within the same refresh cycle RC. This makes it possible to synchronize timing of start of the refresh cycle RC and timing of start of the sampling cycle SC with each other easily.

Like in the third embodiment, the control portion 430 is configured to generate the average signal S (S11, S12, and S13 shown in FIG. 11) by taking a average of a plurality of detection signals from a detection signal acquired first to a detection signal acquired last within one refresh cycle RC. This allows all the average signals S (S11 to S13) to cover the same number of detection signals (the same number of detecting sections).

More specifically, as shown in FIG. 11, the average signal S11 includes five detection signals from a detection signal acquired in a detecting section (1) to a detection signal acquired in a detecting section (5). The average signal S12 includes five detection signals from a detection signal acquired in a detecting section (6) to a detection signal acquired in a detecting section (10). The average signal S12 includes five detection signals from a detection signal acquired in a detecting section (11) to a detection signal acquired in a detecting section (15). As described above, in the fifth embodiment, a photoacoustic image can be generated while all the average signals S (S11 to S13) cover the same number of detecting sections (the same number of detection signals).

The configuration of the fifth embodiment is the same in the other respects as that of the third embodiment.

The fifth embodiment achieves the following effect.

As described above, in the fifth embodiment, the control portion 430 is provided that generates a photoacoustic image to be displayed on the display portion 240 based on the average signal S. By doing so, in the fifth embodiment, all acquired detection signals can be used efficiently for generating a photoacoustic image, like in the third embodiment.

As described above, in the fifth embodiment, the control portion 430 is configured to synchronize timing of start of the refresh cycle RC and timing of start of the sampling cycle SC with each other for each refresh cycle RC. Thus, non-uniformity of the number of detection signals between the refresh cycles RC can be less likely to occur in each refresh cycle RC. In this way, the number of detection signals to form the average signal S can be less likely to vary between the refresh cycles RC, making it possible to reduce the likelihood of the occurrence of a difference in an image quality between individual photoacoustic images to be generated.

As described above, in the fifth embodiment, the control portion 430 is configured to synchronize timing of start of the refresh cycle RC and timing of start of the sampling cycle SC with each other by changing the length of the last sampling cycle SC within the refresh cycle RC. Thus, timing of start of the refresh cycle RC and timing of start of the sampling cycle SC can be synchronized with each other only by changing the length of the last sampling cycle SC within the sampling cycle SC. As a result, timing of start of the refresh rate cycle RC and timing of start of the sampling cycle SC can be synchronized with each other easily.

The other effect of the fifth embodiment is the same as that achieved by the third embodiment.

The embodiments disclosed herein must be considered to be illustrative in all aspects and not restrictive. The range of the present invention is understood not by the above description of the embodiments but by the scope of claims for patent. All changes (modifications) within the meaning and range equivalent to the scope of claims for patent are to be embraced.

For example, in the example shown in the above-described first and second embodiments, during averaging processing by taking of a moving average (average), the control portion 30 (130) takes a moving average (average) of detection signals with the number of additions that makes a value (time) obtained by multiplying the time of the sampling cycle SC and the number of additions during taking of a moving average (average) exceed the time of the refresh cycle RC. However, this is not to limit the present invention. In the present invention, as shown in FIG. 3, during averaging processing by taking of a moving average (average), a control portion may also take a moving average (average) of detection signals with the number of additions that makes a value (time) obtained by multiplying the time of a sampling cycle and the number of additions during taking of a moving average (average) equal to the time of a refresh cycle or less. In this case, a smaller number of additions achieves a corresponding saving of the volume of a storage portion (memory), compared to the case of FIG. 4.

In the example shown in the above-described first embodiment, the control portion 30 takes a moving average of detection signals with the number of additions that makes a value (time) obtained by multiplying the time of the sampling cycle SC and the number of additions during taking of a moving average about 125 msec or less. However, this is not to limit the present invention. In the present invention, a control portion may also take a moving average of detection signals with the number of additions that makes a value (time) obtained by multiplying the time of a sampling cycle and the number of additions during taking of a moving average exceed 125 msec.

In the example shown in the above-described first and second embodiments, several detecting sections exist within one refresh cycle RC. However, this is not to limit the present invention. In the invention, a refresh cycle covering only one detecting section may be present.

In the example shown in the above-described third and fourth embodiments, a photoacoustic image is generated through averaging processing executed by taking a moving average (average) of the average signals S. However, this is not to limit the present invention. In the invention, a photoacoustic image may also be generated through processing other than averaging processing such as taking a moving average or a average.

In the example shown in the above-described third and fourth embodiments, a photoacoustic image is generated by acquiring the average signals S as a plurality of units and using the acquired average signals S as the plurality of units as a basis. However, this is not to limit the present invention. In the invention, if the average signal S includes detection signals of a certain number or more, for example, a photoacoustic image may also be generated based on a average signal as one unit without acquiring the average signals S as a plurality of units.

In the example shown in the above-described first to fifth embodiments, an arithmetic average is taken by taking a average or a moving average. However, this is not to limit the present invention. In the invention, an arithmetic average may be different from a average or a moving average. For example, an arithmetic average may be taken by weighted averaging (weighting averaging) of taking an average of detection signals each given a weight.

### Reference Sings List

10 Semiconductor light-emitting element light source portion
11b, 211b Semiconductor light-emitting element
20, 220 Detecting portion
30, 130, 230, 330, 430 Control portion
40, 240 Display portion
40a, 240a Screen
100, 200, 300, 400, 500 Photoacoustic imaging apparatus
210 Light source portion (semiconductor light-emitting element light source portion)
RC Refresh cycle (cycle of certain refresh rate)
SC Sampling cycle
S, S1, S2, S3, S11, S12, S13 Average signal

## Claims

1. A photoacoustic imaging apparatus comprising:
a semiconductor light-emitting element light source portion (10, 210);
a detecting portion (20, 220) that detects an acoustic wave (AW) generated from a detection target (Q) in a test object (P) when the detection target absorbs light emitted from the semiconductor light-emitting element light source portion and outputs a detection signal;
a display portion (40, 240) having a screen (40a, 240a) to be updated at a certain refresh rate (RC) and on which an image is to be displayed; and
a control portion (30, 130, 230, 330, 430) that makes the semiconductor light-emitting element light source portion emit light in a sampling cycle (SC) shorter than a cycle of the certain refresh rate, acquires the detection signal in the sampling cycle, and generates an image to be displayed on the display portion using the detection signal detected in the sampling cycle.

2. The photoacoustic imaging apparatus according to claim 1, wherein
the control portion is configured to generate an image to be displayed on the display portion by taking an arithmetic average of the detection signals detected in the sampling cycles.

3. The photoacoustic imaging apparatus according to claim 2, wherein
the sampling cycle has a length that allows the semiconductor light-emitting element light source portion to emit light several times while allowing the detection signal to be acquired several times within the cycle of the certain refresh rate.

4. The photoacoustic imaging apparatus according to claim 2, wherein
the control portion is configured to generate an image to be displayed on the display portion by taking an arithmetic average of the detection signals with the number of additions that makes a value obtained by multiplying the sampling cycle and the number of additions during taking of an arithmetic average exceed the cycle of the certain refresh rate.

5. The photoacoustic imaging apparatus according to claim 2, wherein
the control portion is configured to generate an image to be displayed on the display portion by taking a moving average as an arithmetic average.

6. The photoacoustic imaging apparatus according to claim 5, wherein
the control portion is configured to take a moving average each time the detection signal is acquired.

7. The photoacoustic imaging apparatus according to claim 2, wherein
the control portion is configured to generate an image to be displayed on the display portion by taking a average as an arithmetic average.

8. The photoacoustic imaging apparatus according to claim 2, wherein
the control portion is configured to generate an image to be displayed on the display portion by taking an arithmetic average of the detection signals with the number of additions that makes a value obtained by multiplying the sampling cycle and the number of additions during taking of an arithmetic average 125 msec or less.

9. The photoacoustic imaging apparatus according to claim 1, wherein
the control portion is configured to generate a average signal (S, S1, S2, S3, S11, S12, S13) by taking a average of detection signals from a detection signal acquired first to a detection signal acquired last within the cycle of the certain refresh rate and to generate an image to be displayed on the display portion based on the average signal.

10. The photoacoustic imaging apparatus according to claim 9, wherein
the control portion is configured to generate an image to be displayed on the display portion by defining the average signal generated by taking a average as one unit and acquiring average signals as a plurality of units, and by taking an arithmetic average of the acquired average signals as the plurality of units.

11. The photoacoustic imaging apparatus according to claim 10, wherein
the control portion is configured to generate an image to be displayed on the display portion by taking a moving average as an arithmetic average.

12. The photoacoustic imaging apparatus according to claim 11, wherein
the control portion is configured to take a moving average each time the average signal is acquired.

13. The photoacoustic imaging apparatus according to claim 10, wherein
the control portion is configured to generate an image to be displayed on the display portion by taking a average as an arithmetic average.

14. The photoacoustic imaging apparatus according to claim 10, wherein
the control portion is configured to acquire the average signals as a plurality of units in such a manner that a value obtained by multiplying the sampling cycle and the number of detection signals to be subjected to averaging processing by taking of an arithmetic average is 125 msec or less.

15. The photoacoustic imaging apparatus according to claim 9, wherein
the control portion is configured to acquire the detection signal in a certain sampling cycle, and
the control portion is configured to synchronize timing of start of the cycle of the certain refresh rate and timing of start of the certain sampling cycle with each other for each cycle of the certain refresh rate.

16. The photoacoustic imaging apparatus according to claim 15, wherein
the control portion is configured to synchronize timing of start of the cycle of the certain refresh rate and timing of start of the certain sampling cycle with each other by changing the length of a last sampling cycle within the certain refresh cycle.

17. The photoacoustic imaging apparatus according to claim 9, wherein
the control portion is configured to define the average signal generated by taking a average as one unit and acquire the average signal as one unit or acquire the average signals as a plurality of units in such a manner that the number of the detection signals reaches a certain number or more, and to generate an image to be displayed on the display portion based on the acquired average signal as one unit or the acquired average signals as the plurality of units.

18. The photoacoustic imaging apparatus according to claim 1, wherein
the semiconductor light-emitting element light source portion includes a light-emitting diode element as the semiconductor light-emitting element.

19. The photoacoustic imaging apparatus according to claim 1, wherein
the semiconductor light-emitting element light source portion includes a semiconductor laser element as the semiconductor light-emitting element.

20. The photoacoustic imaging apparatus according to claim 1, wherein
the semiconductor light-emitting element light source portion includes an organic light-emitting diode element as the semiconductor light-emitting element.
